# EUROPEAN PATENT APPLICATION

(11) **EP 4 830 780 A2**
(43) Date of publication of application: **28.10.2026**
(21) Application number: 26204016.5
(22) Date of filing: 28.06.2024
(51) Int. Cl.: A61N 5/10

(54) **ELECTRON BEAM OUTPUT DEVICE FOR MEDICAL USE**

(30) Priority: 30.06.2023 KR 20230084840; 10.06.2024 KR 20240074851
(62) Document number(s) of the earlier application(s) in accordance with Art. 76 EPC: 26150696.8 / 4 699 651; 24185289.6 / 4 483 948
(71) Applicant: RADEXEL INC., Gangwon-do 24437 (KR)
(72) Inventor: JUNG, Nuri Hyun, 24363 Chuncheon-si (KR); LEE, Minsik, 24363 Chuncheon-si (KR); BAHNG, Jungbae, 30141 Sejong-si (KR)
(74) Representative: HGF

(57) **Abstract**

The present disclosure relates to an electron beam output device for medical use. The electron beam output device includes at least: an electron beam generator that generates an electron beam having a pencil beam shape; an electron beam accelerator that receives the electron beam generated by the electron beam generator and accelerates the received electron beam; a catheter, through which the electron beam received from the electron beam accelerator passes, the catheter is placed to be directed toward a target site, or is configured to enter an inner passage of the target site; and a first magnetic field generator that is provided in the catheter and generates a magnetic field that refracts the electron beam that is outputted from the catheter.

## Description

### [TECHNICAL FIELD]

Embodiments of the inventive concept described herein relate to an electron beam output device for medical use.

### [BACKGROUND ART]

A medical radiation output device refers to a device that outputs radiation to a target site for the treatment of the target site. Here, the target site may be a lesion tissue, and the lesion tissue may include benign tumor tissue and malignant tumor tissue. Moreover, the radiation may be output to the target site, thereby delaying or destroying the growth of the target site. For example, the radiation may include X-rays, electron beams, protons, and carbon ions.

Medical radiation output devices may be categorized into medical X-ray output devices, medical electron beam output devices, medical proton output devices, and medical carbon ion output devices based on the type of radiation output directed to the target site. Among the medical radiation output devices, the medical electron beam output device is capable of focusing electron beams onto a surface of the target site and a certain depth of the underlying tissue. The medical electron beam output device is widely used because the medical electron beam output device may minimize the dose transmitted to normal tissues surrounding the target site or deeper. Here, the normal site may be a normal tissue.

In a conventional medical electron beam output device, accelerated electron beams are scattered and sprayed toward a wide area in front. As the remaining electron beams other than the electron beams face the target site are absorbed by an applicator, electron beams are irradiated to the target site with a shape similar to that of the target site.

However, because the conventional medical electron beam output device may output only electron beams having only the scattered and diffused shape (a cone beam shape) in front, an output area of the electron beams is restricted to the forward-facing plane area corresponding to a direction in which the electron beams are accelerated.

Accordingly, when the conventional medical electron beam output device outputs electron beams to a target site located in the space inside a living body by passing the electron beams through a narrow entryway inside the living body, when the conventional medical electron beam output device outputs electron beams to a side site of the target site, and when the conventional medical electron beam output device divides the output area of electron beams into several areas and outputs electron beams of different intensities to the target site, it is difficult for the conventional medical electron beam output device to deliver electron beams to the target site as much as the targeted dose.

In detail, the related art, when electron beams are output to a target site located in the living body's internal space through a narrow entryway, to facilitate passage through this entryway, an applicator that absorbs electron beams is installed on lateral sides of the entryway. Consequently, the approach limits electron beam delivery only to a limited area located at the front of the entryway within the target site, resulting in a deficiency in the therapeutic efficacy at the target area.

Furthermore, in the related art, when the applicator that absorb electron beams are not installed on the lateral sides of the narrow entryway inside the living body, the electron beams are irradiated to a normal site in addition to the target site. Accordingly, side effects may occur in the normal site.

Besides, in the related art, sizes of lesions and progression degrees of lesions are different from one another depending on the zone of the target site. Even though the dose of the electron beam output to each zone of the target site needs to be adjusted, it is impossible to adjust the dose of electron beams output to the target site.

For example, electron beams are outputted to a target site located in the space inside the living body by passing the electron beams through the narrow entryway inside the living body for the purpose of a minimally invasive surgery using laparoscopy or thoracoscopy, breast conserving surgery for breast cancer, or treatment for tumors of oral cavity, nasopharynx, pharynx, larynx, esophagus, airway, anus, rectum, or large intestine. The intensity of electron beams is adjusted depending on the zone of the target site for the purpose of a treatment in which a high dose is irradiated to an area visually identified in various tumors and a low dose is irradiated to an area where tumors are expected to be identified through a microscope.

Moreover, in the related art, because accelerated electron beams pass through an applicator and are scattered or absorbed in various parts before reaching the target site, the output efficiency of electron beams may decrease. In detail, in the related art, due to the absorption of a scattering process of accelerated electron beams, the applicator absorption of electron beams, and the absorption in the air while electron beams reach a living body part from the applicator of electron beams, a significant portion of the accelerated electron beams has not reached the target site. As a result, in the related art, a low-power device fails to perform a rapid treatment, and a high-power device is required for the rapid treatment.

### [DETAILED DESCRIPTION OF THE INVENTION]

### [TECHINICAL PROBLEM]

Embodiments of the inventive concept provide an electron beam output device for medical use that allows electron beams to pass through a narrow area and to be irradiated to a target site by adjusting a direction of an accelerated electron beam so as to be output, and allows electron beams to be irradiated not only to a linear output area in a direction in which the electron beam is accelerated, but also to a lateral area outside the linear output area.

Embodiments of the inventive concept provide the electron beam output device that allows electron beams having an appropriate dose to be irradiated depending on a zone of the target site by adjusting the intensity of the electron beam so as to be output.

Embodiments of the inventive concept provide the electron beam output device that minimizes the loss of electron beams to have high output efficiency by outputting an electron beam having a pencil beam shape for minimizing the scattering and diffusion of the electron beams.

Problems to be solved by the inventive concept are not limited to the problems mentioned above, and other problems not mentioned will be clearly understood by those skilled in the art from the following description.

### [TECHNICAL SOLUTION]

According to an embodiment, an electron beam output device includes an electron beam generator that generates an electron beam having a pencil beam shape, an electron beam accelerator that receives the electron beam generated by the electron beam generator and accelerate the received electron beam, a catheter, through which the electron beam received from the electron beam accelerator passes, wherein the catheter is placed to be directed toward a target site, or enters an inner passage of the target site, and a first magnetic field generator provided in the catheter and generates a magnetic field that refracts the electron beam that is outputted from the catheter.

Other details according to some embodiments are included in the detailed description and drawings.

### [ADVANTAGEOUS EFFECTS OF THE INVENTION]

According to some embodiments, an electron beam output device may allow electron beams to pass through a narrow area and to be irradiated to a target site by adjusting and outputting a direction of an accelerated electron beam, and may output electron beams not only to a linear output area in a direction in which the electron beam is accelerated, but also to a lateral area outside the linear output area.

Moreover, according to some embodiments, an electron beam output device may output an electron beam having an appropriate dose to each zone of the target site by adjusting and outputting the intensity of the electron beam.

Furthermore, according to some embodiments, an electron beam output device may not require a conventional electron scattering unit and a conventional applicator, thereby minimizing the loss of the electron beam generated by the electron beam generator, reducing power consumption in the electron beam generator, and improving the intensity of the electron beam generated from the electron beam generator.

Effects of the inventive concept are not limited to the effects mentioned above, and other effects not mentioned will be clearly understood by those skilled in the art from the following description.

### [DESCRIPTION OF THE DRAWINGS]

FIG. 1 is a schematic diagram illustrating an electron beam output device according to some embodiments;
FIG. 2 is a schematic diagram illustrating a controller of an electron beam output device according to some embodiments;
FIGS. 3A and 3B are schematic diagrams illustrating an electron beam output device according to some embodiments;
FIGS. 4A to 4C are a block diagram illustrating an electron beam output device according to some embodiments;
FIG. 4D is a schematic diagram illustrating a second magnetic field generator of an electron beam output device according to some embodiments.
FIG. 4E is a schematic diagram illustrating a second magnetic field generator of an electron beam output device according to some other embodiments.
FIG. 5 is a side view showing an example of a first magnetic field generator of an electron beam output device according to some embodiments;
FIG. 6A is a perspective view showing an example of a first magnetic field generator of an electron beam output device according to some embodiments;
FIG. 6B is a cross-sectional view showing an example of a first magnetic field generator of an electron beam output device according to some embodiments;
FIG. 7 is a schematic diagram illustrating an example of a state where an electron beam refracted by a first magnetic field generator of an electron beam output device is outputted to a target site, according some embodiments;
FIGS. 8A to 8D are schematic diagrams illustrating another example of a state where an electron beam refracted by a first magnetic field generator of an electron beam output device is outputted to a target site, according some embodiments;
FIGS. 9A to 9D are schematic diagrams illustrating another example of a state where an electron beam refracted by a first magnetic field generator of an electron beam output device is outputted to a target site, according to some embodiments;
FIG. 10A is a side view illustrating a shape of an electron beam refracted by a first magnetic field generator of an electron beam output device, according to some embodiments; and
FIG. 10B is a rear view illustrating a shape of an electron beam refracted by a first magnetic field generator of an electron beam output device, according to some embodiments.

### [DETAILED DESCRIPTION]

The above and other aspects, features and advantages of the inventive concept will become apparent from embodiments to be described in detail in conjunction with the accompanying drawings. The inventive concept, however, may be embodied in various different forms, and should not be construed as being limited only to the illustrated embodiments. Rather, these embodiments are provided as examples so that the inventive concept will be thorough and complete, and will fully convey the scope of the inventive concept to those skilled in the art. The inventive concept may be defined by the scope of the claims.

The terms used herein are provided to describe embodiments, not intended to limit the inventive concept. In the specification, the singular forms include plural forms unless particularly mentioned. The terms "comprises" and/or "comprising" used herein do not exclude the presence or addition of one or more other components, in addition to the aforementioned components. The same reference numerals denote the same components throughout the specification. As used herein, the term "and/or" includes each of the associated components and all combinations of one or more of the associated components. It will be understood that, although the terms "first", "second", etc., may be used herein to describe various components, these components should not be limited by these terms. These terms are only used to distinguish one component from another component. Thus, a first component that is discussed below could be termed a second component without departing from the technical idea of the inventive concept.

Unless otherwise defined, all terms (including technical and scientific terms) used herein have the same meaning as commonly understood by those skilled in the art to which the inventive concept pertains. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein.

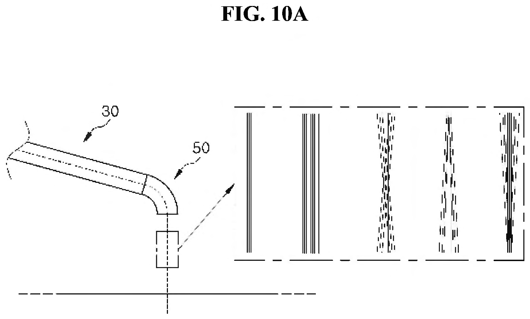

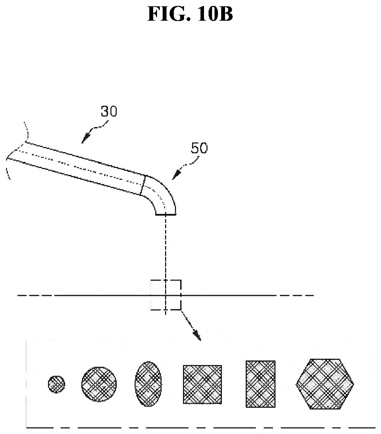

A general conventional medical electron beam output device uses electron beams that are scattered in a wide range in a traveling direction of an electron beam by using a scattering foil or the like. In some embodiments, a term of a pencil beam may mean an electron beam that is not scattered. For example, as shown in FIG. 10A, a lateral shape of the pencil beam may have various shapes such as a line shape, a shape in which a horizontal width gradually become narrower from the top to the center and then the horizontal width gradually widens from the center to the top, a shape in which a horizontal width gradually widens from the top to the center, and a shape in which a horizontal width gradually become narrower from the top to the center. For another example, as shown in FIG. 10B, a back shape of the pencil beam may have various shapes such as a point shape, a circular shape, an elliptical shape, a square shape, and a hexagon shape.

According to some embodiments, the electron beam output device for medical use may output an electron beam to a target site of the living body by adjusting the direction and intensity of the electron beam, and may be used for the following purposes.

For example, according to some embodiments, the electron beam output device for medical use may be used to provide electron beams required for endoscopic surgery and procedures. In this case, when a catheter 30 or an endoscope is made of a rigid material, electron beams may be provided to the cranial cavity, abdominal cavity, thoracic cavity, pelvic cavity, nasal cavity, paranasal sinuses, ears, oral cavity, pharynx, larynx, trachea, esophagus, rectum, anus, bladder, joints, spine/spinal cord, or the like. Moreover, when the catheter 30 or the endoscope is made of a soft material, electron beams may be provided to esophagus, stomach, duodenum, trachea, bronchus, large intestine, rectum, and bladder, into each of which the catheter 30 is easily entered.

For example, according to some embodiments, the electron beam output device for medical use may be used to provide electron beams required for robotic endoscopic surgery.

For example, according to some embodiments, the electron beam output device for medical use may be used to provide electron beams necessary for the skin, breast, eye (conjunctiva, retina, or the like), oral cavity, uterus, cervix, and rectum.

Hereinafter, some embodiments will be described in detail with reference to the accompanying drawings.

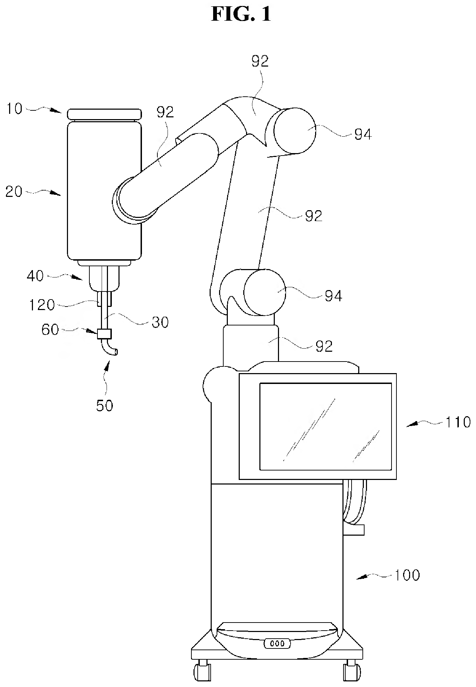

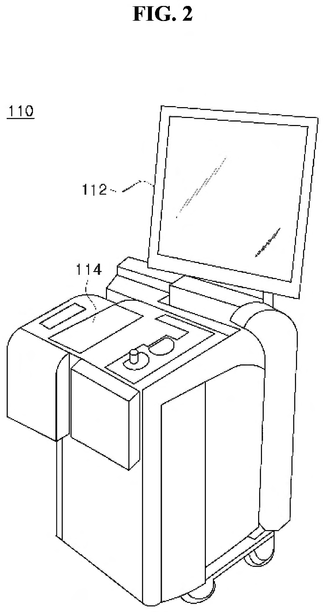

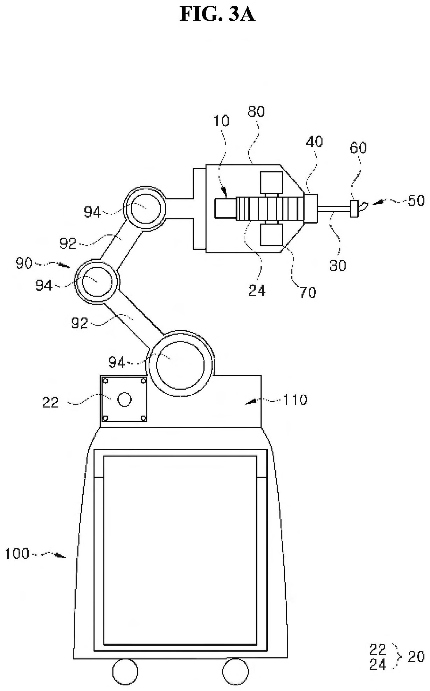

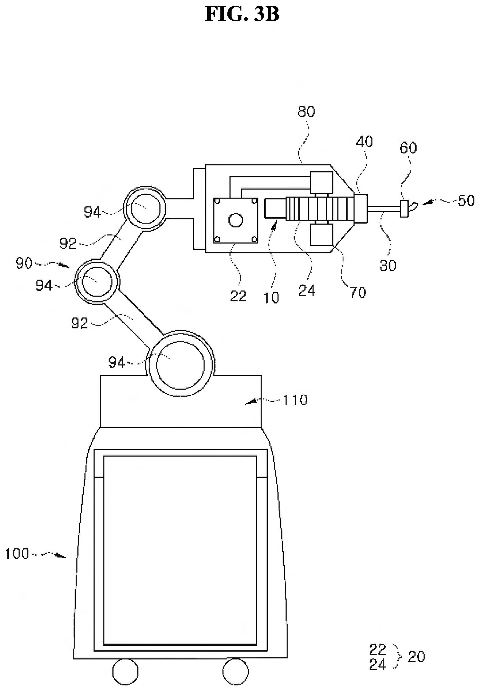

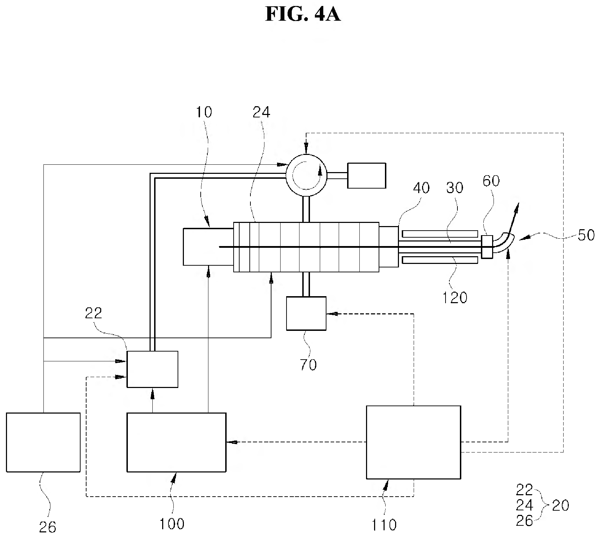

FIG. 1 is a schematic diagram illustrating an electron beam output device for medical use, according to some embodiments. FIG. 2 is a schematic diagram illustrating a controller 110 of an electron beam output device according to some embodiments. FIGS. 3A and 3B are schematic diagrams illustrating an electron beam output device according to some embodiments. FIGS. 4A to 4C are a block diagram illustrating an electron beam output device according to some embodiments.

As illustrated in FIGS. 1 to 4C, according to some embodiments, the electron beam output device may include an electron beam generator 10, an electron beam accelerator 20, the catheter 30, a catheter driver 40, a first magnetic field generator 50, a first magnetic field generator driver 60, a vacuum pump 70, a shield unit 80, a robotic arm 90, a power supply unit 100, and a controller 110. Here, the electron beam generator 10, the electron beam accelerator 20, the catheter 30, the first magnetic field generator 50, the vacuum pump 70, and the shield unit 80 may correspond to the head of an electron beam output device. Moreover, the robotic arm 90 and the power supply unit 100 may be the body of an electron beam output device. Furthermore, the controller 110 may be a console of an electron beam output device. Here, the head of the electron beam output device may be used to be compatible with a power supply unit of the electron beam output device currently on the market.

The electron beam generator 10 serves to generate an electron beam. The electron beam generator 10 may generate an electron beam by receiving power from the power supply unit 100 to be described later. Besides, the electron beam generated by the electron beam generator 10 may be accelerated by an electric field generated by the electron beam accelerator 20, may pass through the catheter 30 in the form of a pencil beam, may be refracted by a magnetic field formed by the first magnetic field generator 50, and may be output to a target site. In this case, as the electron beam has the pencil beam shape, the size of the magnetic field area required for refraction of the electron beam may be reduced. Accordingly, the first magnetic field generator 50 may be miniaturized. Furthermore, as the first magnetic field generator 50 is capable of being miniaturized, the catheter 30 and the first magnetic field generator 50 may be easily inserted into a narrow inner passage of the target site.

For example, the electron beam generator 10 may generate an electron beam with an intensity of 1 to 50 MeV and, more preferably, may generate an electron beam with an intensity of 1 to 10 MeV. In some embodiments, the electron beam generator 10 may generate an electron beam having a dose rate equal to or higher than 40 Gy/s.

The electron beam generator 10 may operate in a first output mode, in which an electron beam of a first output intensity is generated, or a second output mode in which an electron beam of a second output intensity is generated. Here, the first output intensity may be lower than the second output intensity.

For example, the electron beam generator 10 may operate in only the first output mode. As such, a preheating process may be omitted in the electron beam generator 10 operating in only the first output mode, and the electron beam generator 10 may be miniaturized.

For another example, the electron beam generator 10 may operate in only the second output mode. As such, the electron beam generator 10 operating in only the second output mode may output an electron beam with the second output intensity greater than the first output intensity, thereby shortening a time required to output the electron beam to the target site and exhibiting a FLASH effect. For reference, the FLASH effect may occur when radiation including electron beams is output to the target site at a dose rate of 40 Gy/s or higher. Considering that a general dose rate of a radiation output device is 0.1 Gy/s and a dose rate of a radiation output device is about 10 Gy/s under special conditions, the dose rate for the FLASH effect is 400 times higher than that of general radiation. In a general X-ray output device for medical use that provides a dose rate of 0.1 Gy/s, a dose rate reduction of 99% occurs when accelerated electrons are converted into X-rays by tungsten or the like. The dose rate reduction of 50% may occur in a flattening filter, and the accelerated electrons are spread to a place spaced away from an X-ray generation point by about 100 cm. Finally, the dose rate reduction of 99% may occur. Accordingly, X-rays having a dose rate of about 0.006% are irradiated to the final target site. Accordingly, in an example of the inventive concept, the accelerated electron beam is irradiated to the target site in a state where the loss of accelerated electron beam having a pencil beam is minimized, and thus radiation may be irradiated at a dose rate about 16,000 times higher than the radiation of a general X-ray generating device for medical use. Accordingly, a FLASH effect may occur. Furthermore, the FLASH effect may reduce the damage to normal tissue adjacent to the target site due to the output of the electron beam, and may shorten a treatment time by outputting the electron beam to the target site.

The electron beam accelerator 20 may serve to receive the electron beam from the electron beam generator 10 and accelerate the received electron beam. For example, the electron beam accelerator 20 may be connected to a part where an electron beam generated from the electron beam generator 10 is irradiated. For example, when the electron beam accelerator 20 is connected to the electron beam generator 10 operating in only the first output mode, the electron beam accelerator 20 may be miniaturized.

The electron beam accelerator 20 may include a radio frequency (RF) generator 22, an accelerator tube 24, an accelerator tube cooler 26, an RF circulator, and an RF load (or a dummy load).

The RF generator 22 may generate a high-frequency wave. The RF generator 22 may generate a high-frequency wave by receiving power from the power supply unit 100 to be described later.

For example, when RF generator 22 is connected to the electron beam generator 10 operating in only the first output mode, the RF generator 22 may be miniaturized.

For example, the RF generator 22 may be built into the power supply unit 100 (see FIG. 3A). As another example, the RF generator 22 may be embedded in the shield unit 80 to be described later.

The accelerator tube 24 may accelerate the electron beam received from the electron beam generator 10 by forming an electric field by the high-frequency wave transmitted from the RF generator 22. In detail, the electric field formed in the accelerator tube 24 may push the electron beam delivered to the accelerator tube 24 toward the catheter 30.

The RF circulator delivers the high-frequency wave generated from the RF generator 22 to the accelerator tube 24 in one direction.

The dummy load absorbs and removes unnecessary high-frequency waves that are present around the accelerator tube 24.

The accelerator tube cooler 26 may cool the accelerator tube 24. For example, the accelerator tube cooler may cool the accelerator tube 24 by using at least one of a water cooling method, an air cooling method, and an oil cooling method.

For example, when the accelerator tube cooler 26 is connected to the electron beam generator 10 operating in only the first output mode, the accelerator tube cooler 26 may cool the accelerator tube 24 with a relatively small flow rate as the entire device is miniaturized and the temperature rise rate of an accelerator tube decreases.

The catheter 30 may pass the electron beam received from the electron beam accelerator 20, and may be disposed to be directed toward a target site or may enter an inner passage of the target site.

For example, as a channel is formed inside the catheter 30, the electron beam transmitted from the electron beam accelerator 20 may pass through the channel. At this time, to reduce the scattering of the electron beam, a vacuum may be formed in the channel of the catheter 30 by a vacuum unit to be described later. Moreover, to reduce the scattering of the electron beam, the channel of the catheter 30 may be filled with helium. Also, to reduce the scattering of the electron beam, the channel of the catheter 30 may be filled with air. In this way, the channel of the catheter 30, in which vacuum is formed or helium is filled, and there is no scattering foil between the accelerator tube 24 and the catheter 30. Therefore, the loss of the electron beam passing through the accelerator tube 24 and the catheter 30 is minimized, and accordingly, the electron beam having a pencil beam shape may be output.

For example, the catheter 30 may have a length of 10 to 50 cm and, more preferably, may have a length of 20 to 30 cm. Furthermore, the catheter 30 may have an outer diameter of 0.2 to 5 cm, more preferably 0.3 to 3 cm. If the length of the catheter 30 is less than 10 cm, it is difficult for the catheter 30 to easily enter the incision in the human body or to access it in various directions. If the length of the catheter 30 is greater than 50 cm, it is difficult to use the catheter 30 in a limited space such as an operating room. Therefore, the length of the catheter 30 is preferably 10 to 50 cm. Meanwhile, if the outer diameter of the catheter 30 is less than 0.2 cm, the catheter 30 cannot transfer an electron beam having a pencil beam shape. If the outer diameter of the catheter 30 is greater than 5 cm, it is difficult to enter the incision in the human body. Therefore, the outer diameter of the catheter 30 is preferably 0.2 to 5 cm.

For example, as the catheter 30 is made of a flexible material, the catheter 30 may bend along the inner passage of the target site when the inner passage of the target site is entered, thereby easily entering the inner passage of the target site.

For example, as the catheter 30 is made of plastic, it is possible to reduce the amount of secondary X-rays generated as some electron beams collide with the catheter 30.

For example, the catheter 30 may be detachably connected to the accelerator tube 24. In this example, the catheter 30 may be screwed to the accelerator tube 24.

For example, the catheter 30 may be integrally connected to the accelerator tube 24. In this example, the catheter 30 may be joined to the accelerator tube 24. Besides, the catheter 30 may be welded to the accelerator tube 24.

For example, the catheter 30 may be connected such that the catheter 30 relatively rotates with respect to the accelerator tube 24, and may relatively rotate with respect to the accelerator tube 24 by the catheter driver 40 to be described later.

For example, the length of the catheter 30 may be adjustable, and the length thereof may be adjusted by the catheter driver 40 to be described later. Here, the catheter 30 may be made of a flexible material such that the length of the catheter 30 is adjustable. Moreover, the catheter 30 may have a bellows shape such that the length of the catheter 30 is adjustable. The length of the catheter 30 may be adjusted by the catheter driver 40 to be described later.

The catheter driver 40 may relatively rotate the catheter 30 about the accelerator tube 24, and may move a specific part of the catheter 30 in a direction closer to the accelerator tube 24 or in a direction away from the accelerator tube 24 such that the length of the catheter 30 is adjusted. For example, the catheter driver 40 may relatively rotate the catheter 30 about the accelerator tube 24 in at least one direction of a left-right direction, a front-back direction, and an up-down direction. Furthermore, while being fixed at a specific site of the catheter 30, the catheter driver 40 may move the catheter 30 in at least one direction of a left-right direction, a front-back direction, and an up-down direction.

The first magnetic field generator 50 may be provided in the catheter 30 to generate a magnetic field that refracts an electron beam passing through the catheter 30 or an electron beam output from the catheter 30. In this way, the electron beam refracted by the first magnetic field generator 50 may be output to the target site. In some embodiments, the first magnetic field generator 50 may be provided at a distal end portion of the catheter 30. With this configuration, the magnetic field generated by the first magnetic field generator 50 refracts the electron beam that has completely passed through the catheter 30. Accordingly, the electron beam that has completely passed through the catheter 30 is refracted with various refraction angles at the distal end of the catheter 30 by the magnetic field generated by the first magnetic field generator 50. In this way, in some embodiments, while the end of the catheter 30 is fixed to a specific depth of the incision in the human body, the electron beam that has completely passed through the catheter 30 is refracted with various refraction angles at the distal end of the catheter 30 by the magnetic field generated by the first magnetic field generator 50, and thus, the electron beam can be directed to various points on the side of the incision in the human body.

For example, the first magnetic field generator 50 may include a plurality of magnetic bodies 52, 54 arranged along the circumference of the distal end portion of the catheter 30. In other words, the plurality of magnetic bodies may be arranged in a ring shape around the distal end portion of the catheter 30. A magnetic field that refracts an electron beam passing through the catheter 30 or output from the catheter 30 may be formed in a space surrounded by the plurality of magnetic bodies.

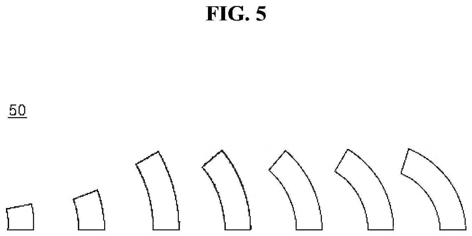

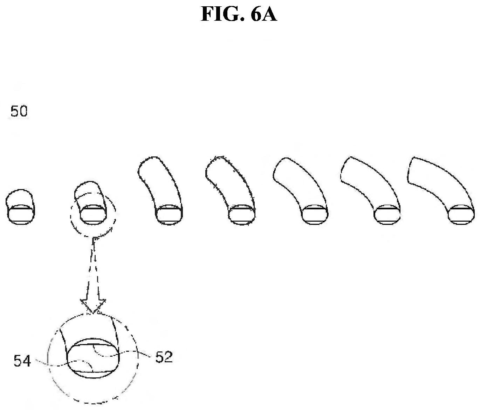

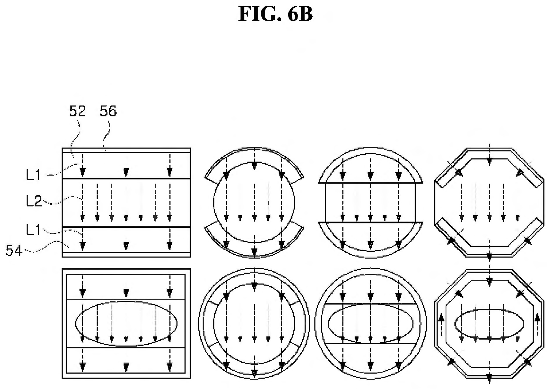

FIG. 5 is a side view showing an example of the first magnetic field generator 50 of an electron beam output device, according to some embodiments. FIG. 6A is a perspective view showing an example of the first magnetic field generator 50 of an electron beam output device, according to some embodiments. FIG. 6B is a cross-sectional view showing an example of a first magnetic field generator of an electron beam output device, according to some embodiments.

Referring to FIGS. 5 to 6B, the first magnetic field generator 50 may include a first magnetic body 52 and a second magnetic body 54, which are disposed to be directed toward each other at the distal end portion of the catheter 30. A magnetic field that refracts an electron beam passing through the catheter 30 or output from the catheter 30 may be formed between the first magnetic body 52 and the second magnetic body 54.

For example, the first magnetic body 52 and the second magnetic body 54 are permanent magnets, and the permanent magnets may have an arc-shaped cross-section. As the arc-shaped magnetic field is formed between the first magnetic body 52 and the second magnetic body 54, an electron beam passing through the catheter 30 or output from the catheter 30 may be refracted.

For another example, when the first magnetic body 52 and the second magnetic body 54 are permanent magnets, an angle at which an electron beam is refracted by the magnetic field formed between the first magnetic body 52 and the second magnetic body 54 may be adjusted by adjusting locations of the first magnetic body 52 and the second magnetic body 54 and adjusting a distance between the first magnetic body 52 and the second magnetic body 54. In detail, as the first magnetic body 52 and the second magnetic body 54 are close to each other, the intensity of a magnetic field between the first magnetic body 52 and the second magnetic body 54 may be increased. Moreover, as the first magnetic body 52 and the second magnetic body 54 move away from each other, the intensity of a magnetic field between the first magnetic body 52 and the second magnetic body 54 may be reduced. In the meantime, the locations of the first magnetic body 52 and the second magnetic body 54 and the distance between the first magnetic body 52 and the second magnetic body 54 may be adjusted by the first magnetic field generator driver 60 to be described later.

For another example, when the first magnetic body 52 and the second magnetic body 54 are permanent magnets, the angle at which an electron beam is refracted by the magnetic field formed between the first magnetic body 52 and the second magnetic body 54 may be adjusted depending on the acceleration control of the electron beam accelerated in the electron beam accelerator 20.

Meanwhile, referring to FIGS. 5 and 6A, when the first magnetic body 52 and the second magnetic body 54 are permanent magnets, there are the plurality of first magnetic bodies 52 and the plurality of second magnetic bodies. In addition, the plurality of first magnetic bodies 52 may have different curvature radii and lengths from one another. The plurality of second magnetic bodies 54 may have curvature radii and lengths corresponding to those of the plurality of first magnetic bodies 52, respectively. The angle at which an electron beam is refracted may be adjusted by the magnetic field formed between the first magnetic body 52 and the second magnetic body 54 by selectively placing one of the plurality of first magnetic bodies 52 and one of the plurality of second magnetic bodies 54 at the distal end portion of the catheter 30.

For example, the first magnetic body 52 and the second magnetic body 54 may be pulsed electromagnets. The pulsed electromagnet may generate a magnetic field synchronized with a pulse of an electron beam passing through the catheter 30. As the magnetic field synchronized with the pulse of the electron beam passing through the catheter 30 is formed between the first magnetic body 52 and the second magnetic body 54, the electron beam passing through the catheter 30 or output from the catheter 30 may be refracted. Meanwhile, the intensity of the magnetic field generated from the pulsed electromagnet may be 0.1 to 1.0 Tesla.

For example, referring to FIG. 6B, a magnetic field shield 56 may be provided around the first magnetic field generator 50. As such, the magnetic field generated from the first magnetic field generator 50 is shielded by the magnetic field shield 56. Accordingly, the intensity of the magnetic field generated by the first magnetic field generator 50 may increases; the homogeneity of the magnetic field may be improved; and, the external leakage of the magnetic field may be reduced. For reference, in FIG. 6B, an electron beam passing space may be formed between the first magnetic body 52 and the second magnetic body 54; L1 of FIG. 6B may be a direction of a magnetic field formed by each of the first magnetic body 52 and the second magnetic body 54; and, L2 of FIG. 6 may be a direction of a composite magnetic field obtained by coupling the magnetic field of the first magnetic body 52 and the magnetic field of the second magnetic body 54 Between the first magnetic body 52 and the second magnetic body 54.

For example, a weight for adjusting the weight distribution of the first magnetic field generator 50 may be provided in the first magnetic field generator 50 based on the output direction of an electron beam. In this way, vibration generated during rotation of the first magnetic field generator 50 may be reduced.

The first magnetic field generator 50 may be connected such that the first magnetic field generator 50 relatively rotates with respect to the catheter 30 and relatively moves with respect to the catheter 30, and may relatively rotate with respect to the catheter 30 and may relatively move with respect to the catheter 30 by the first magnetic field generator driver 60 to be described later.

The first magnetic field generator driver 60 may relatively rotate the first magnetic field generator 50 with respect to the catheter 30 and may relatively move the first magnetic field generator 50 with respect to the catheter 30. For example, the first magnetic field generator driver 60 may relatively rotate the first magnetic field generator 50 with respect to the catheter 30 in at least one direction of a left-right direction, a front-back direction, and an up-down direction. Besides, the first magnetic field generator driver 60 may relatively move the first magnetic field generator 50 with respect to the catheter 30 in at least one direction of a left-right direction, a front-back direction, and an up-down direction.

For example, the first magnetic field generator driver 60 may include an actuator and a driving motor. Moreover, the first magnetic field generator driver 60 may be fixed to an external structure such as a wall.

For example, when the first magnetic body 52 and the second magnetic body 54 are permanent magnets, the first magnetic field generator driver 60 may adjust the locations of the first magnetic body 52 and the second magnetic body 54, and may adjust the distance between the first magnetic body 52 and the second magnetic body 54.

The vacuum pump 70 may form a vacuum in at least one of the accelerator tube 24, the catheter 30, and the first magnetic field generator 50. For example, the vacuum pump 70 may be connected to the accelerator tube 24 to form a vacuum only in the accelerator tube 24. In addition, the vacuum pump 70 may be connected to the accelerator tube 24 to form a vacuum between the accelerator tube 24 and the catheter 30. In addition, the vacuum pump may be connected to the accelerator tube 24 to form a vacuum in the accelerator tube 24, the catheter 30 and the first magnetic field generator 50.

The shield unit 80 may surround the electron beam generator 10 and the electron beam accelerator 20 and may be connected to the catheter 30. In addition, the shield unit 80 may shield electron beams leaking from the electron beam generator 10 and the electron beam accelerator 20. The shield unit 80 may prevent radiation (electron beams or X-rays generated secondarily by electron beams) emitted from the electron beam generator 10 and the electron beam accelerator 20 from exposing medical staff or medical devices located around the electron beam generator 10 and the electron beam accelerator 20.

For example, the shield unit 80 may have a structure that encloses only the end part of the electron beam accelerator 20 so as to be miniaturized.

For example, the shield unit 80 may include a partition wall standing on the electron beam generator 10 and the electron beam accelerator 20.

For example, the shield unit 80 may include a gown covering the perimeter of the target site.

The robotic arm 90 may be connected to the electron beam accelerator 20 to move and rotate the electron beam accelerator 20, the catheter 30, and the first magnetic field generator 50. In this case, the robotic arm 90 may be connected to the center of gravity of the electron beam accelerator 20.

For example, the robotic arm 90 may be connected to the shield unit 80 to move and rotate the electron beam accelerator 20, the catheter 30, and the first magnetic field generator 50. In this case, the robotic arm 90 may be connected to the center of one surface directed toward the electron beam accelerator 20 in the shield unit 80.

For example, the robotic arm 90 may move and rotate the electron beam accelerator 20, the catheter 30, and the first magnetic field generator 50 in six axes. In other words, the robotic arm 90 may rotate the electron beam accelerator 20, the catheter 30, and the first magnetic field generator 50 in at least one direction of a left-right direction, a front-back direction, and an up-down direction, and may move the electron beam accelerator 20, the catheter 30 and the first magnetic field generator 50 in at least one direction of a left-right direction, a front-back direction, and an up-down direction.

For example, the robotic arm 90 may include a plurality of segments 92 connected to the electron beam accelerator 20, a plurality of links 94 rotatably connecting the two adjacent segments 92, and a drive motor that is provided in the link 94 and rotates one of the two segments 92 adjacent to each other (see FIG. 1). Here, a linear guide (not shown) for linearly moving the electron beam accelerator 20 may be provided between the electron beam accelerator 20 and the segment 92. The linear guide may include a linear rail coupled to the electron beam accelerator 20, a mobile body coupled to the segment 92 and linearly moving along the linear rail, and an actuator linearly the mobile body.

The power supply unit 100 may supply power to the electron beam generator 10, the electron beam accelerator 20, and the first magnetic field generator 50. For example, the power supply unit 100 may be a capacitor.

The controller 110 may control the electron beam generator 10, the electron beam accelerator 20, the catheter 30, the catheter driver 40, the first magnetic field generator 50, the first magnetic field generator driver 60, the vacuum pump 70, the shield unit 80, the robotic arm 90, and the power supply unit 100. The controller 110 may control the intensity, dose, dose rate and generation timing of the electron beam generated from the electron beam generator 10, and may control the intensity, direction, and generation timing of the magnetic field generated by the first magnetic field generator 50. In addition, the controller 110 may include a display 112 displaying an image of a target site. Moreover, the controller 110 may include a manipulation unit 114 for manual manipulation of the electron beam generator 10, the electron beam accelerator 20, the catheter 30, the catheter driver 40, the first magnetic field generator 50, the first magnetic field generator driver 60, the vacuum pump 70, the shield unit 80, the robotic arm 90, and the power supply unit 100. For example, a manipulation unit may be implemented as a plurality of buttons.

For example, the controller 110 may be integrally connected to the power supply unit 100.

For another example, the controller 110 may not be connected to the power supply unit 100 and may be provided in a separate shielding room where radiation is shielded.

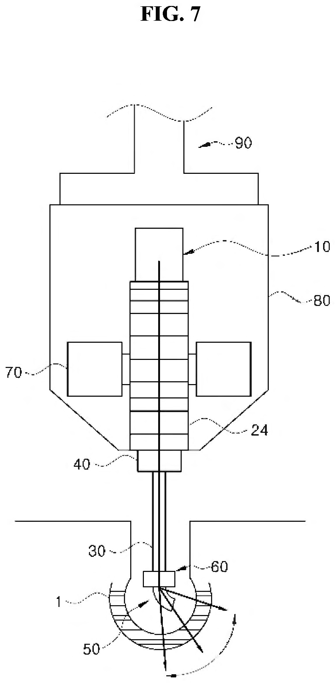

FIG. 7 is a schematic diagram illustrating an example of a state where an electron beam refracted by a first magnetic field generator of an electron beam output device is outputted to a target site, according to some embodiments.

As shown in FIG. 7, the controller 110 may control the catheter driver 40 such that the catheter driver 40 fixes the catheter 30 to the inner passage of a target site 1 having a specific depth, may control the first magnetic field generator driver 60 such that the first magnetic field generator driver 60 adjusts the angle of the first magnetic field generator 50, and may adjust the distribution of the electron beam output from the catheter 30 to the target site. However, FIG. 7 is only an example in which the distribution of electron beams output from the catheter 30 to the target site is adjusted under the control of the controller 110. A method of adjusting the distribution of electron beams output to the target site of the electron beam output device according to some embodiments is not limited thereto.

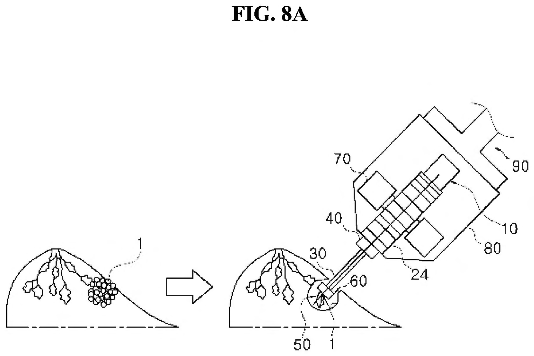

FIGS. 8A to 8D are schematic diagrams illustrating another example of a state where an electron beam refracted by a first magnetic field generator of an electron beam output device is outputted to a target site, according to some embodiments.

As shown in FIGS. 8A to 8D, as the controller 110 controls the catheter driver 40 such that the catheter driver 40 moves and rotates the catheter 30 while the catheter 30 is directed toward a target site, the controller 110 may control the distribution of the electron beam output from the catheter 30 to the target site 1. However, FIGS. 8A to 8D are only examples in each of which the distribution of electron beams output from the catheter 30 to the target site is adjusted under the control of the controller 110. A method of adjusting the distribution of electron beams output to the target site of the electron beam output device according to some embodiments is not limited thereto.

In detail, FIG. 8A shows an example in which the controller 110 controls the catheter driver 40 such that the distribution of electron beams output from the catheter 30 is concentrated to breast cancer tissue when the target site 1 is breast cancer tissue.

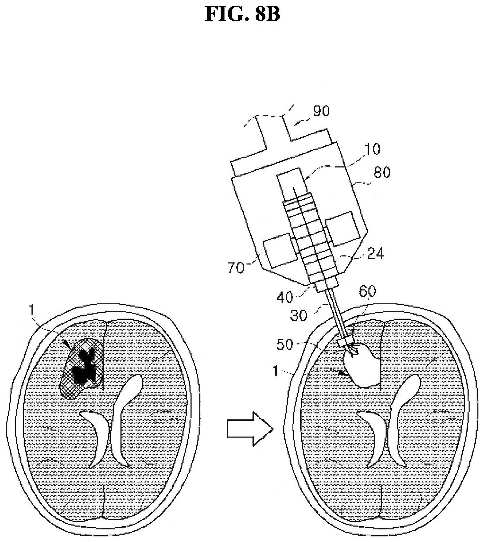

Besides, FIG. 8B shows an example in which the controller 110 controls the catheter driver 40 such that the distribution of electron beams output from the catheter 30 is concentrated to brain tumor tissue when the target site 1 is brain tumor tissue.

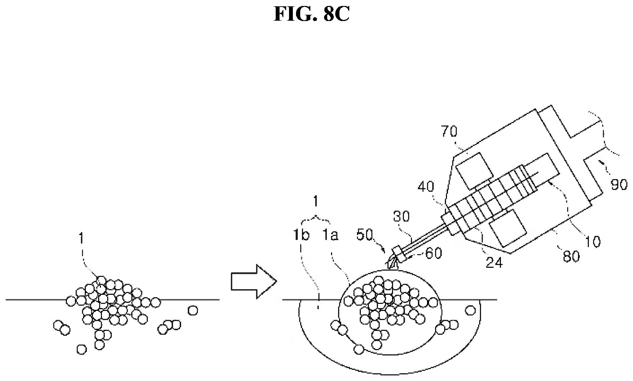

Also, FIG. 8C shows an example in which the controller 110 sequentially operates the electron beam generator 10 in a first output mode and a second output mode, controls the catheter driver 40 such that electron beams output from the catheter 30 are irradiated for a long time to a central zone 1a where skin cancer cells are distributed at a high density, and are irradiated for a shorter time than the central zone 1a to a peripheral zone 1b where skin cancer cells are distributed at a low density, when the target site 1 corresponds to skin cancer tissues. As a result, as a high-dose electron beam is irradiated to the central zone 1a of skin cancer, the skin cancer tissues are effectively destroyed. As a low-dose electron beam is irradiated to the peripheral zone 1b, normal tissues may be protected.

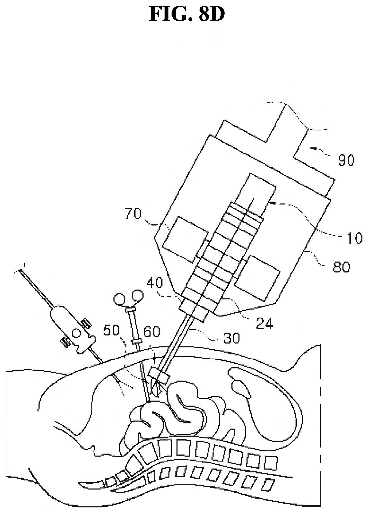

Furthermore, FIG. 8D shows an example in which the controller 110 identifies the location of the target site 1 and the location of a surgical tool taken by an endoscope camera and controls the catheter driver 40 such that the distribution of electron beams output from the catheter 30 is concentrated to the target site 1, when the endoscope camera and a surgical tool are installed in the target site 1.

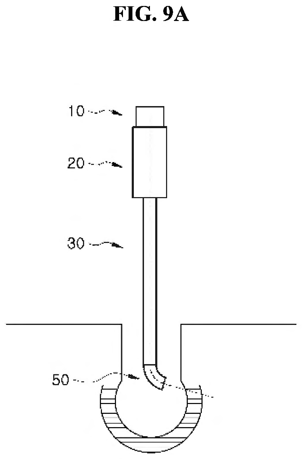

FIGS. 9A to 9D are schematic diagrams illustrating another example of a state where an electron beam refracted by a first magnetic field generator of an electron beam output device is output to a target site, according to some embodiments.

As shown in FIG. 9A, when the distal end portion of the catheter 30 and the first magnetic field generator 50 are directly inserted into the inner passage of the target site 1, electron beams may be output on the side of the target site 1.

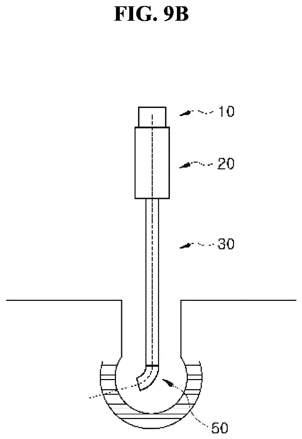

Afterward, as shown in FIG. 9B, when the distal end portion of the catheter 30 and the first magnetic field generator 50 are additionally inserted into the inner passage of the target site 1, electron beams may be output on the lower side of the side of the target site 1 as compared to the illustration FIG. 9A.

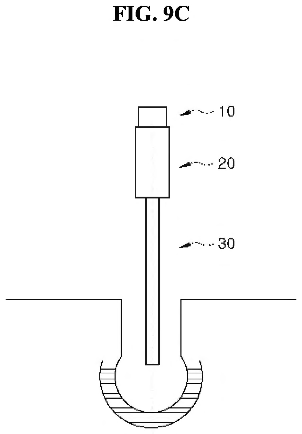

As shown in FIG. 9C, when only the distal end portion of the catheter 30 is inserted into the inner passage of the target site 1, electron beams may be output to the bottom of the target site 1.

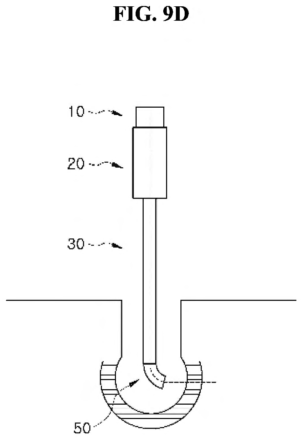

As shown in FIG. 9D, when the first magnetic field generator 50 having a large radius of curvature is fixed to the distal end portion of the catheter 30 as compared to the illustration FIG. 9A, electron beams may be more easily output to the side of the target site 1.

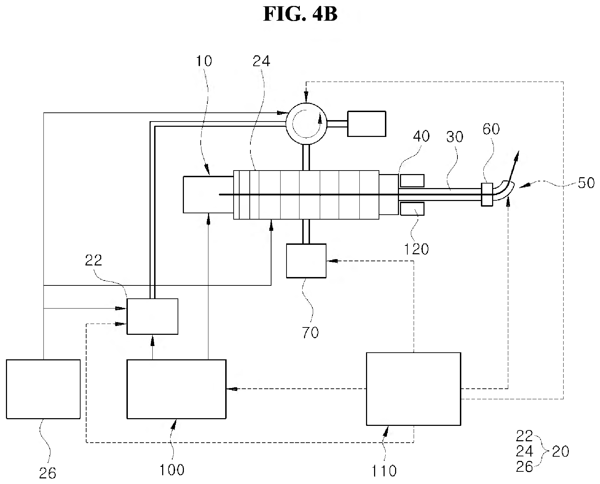

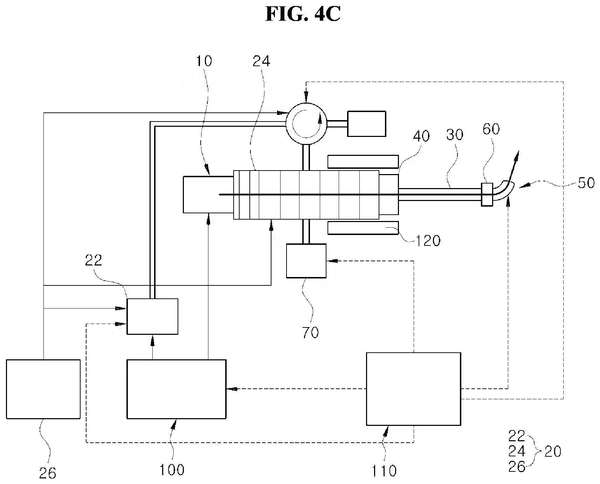

An electron beam output device according to some embodiments may further include a second magnetic field generator 120. Referring to FIG. 4A, in some embodiments, the second magnetic field generator 120 may be provided around the catheter 30. Referring to FIG. 4B, in some embodiments, the second magnetic field generator 120 may be provided around one distal end of the catheter 30 and adjacent to the catheter driver 40. Referring to FIG. 4C, in some embodiments, the second magnetic field generator 120 may be provided around one distal end of the accelerator tube 24 and adjacent to the catheter driver 40.

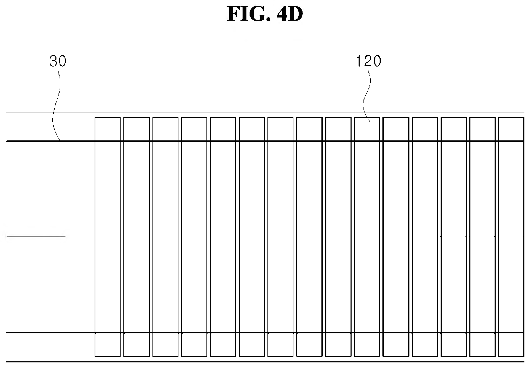

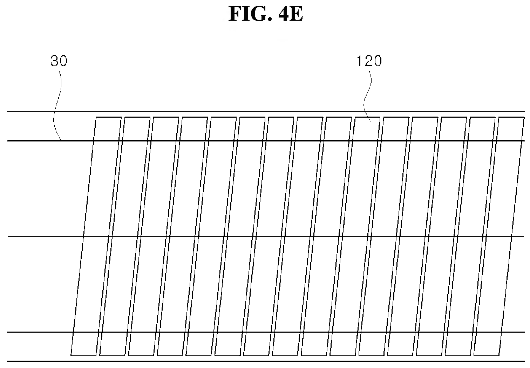

FIG. 4D is a schematic diagram illustrating a second magnetic field generator of an electron beam output device according to some embodiments, and FIG. 4E is a schematic diagram illustrating a second magnetic field generator of an electron beam output device according to some other embodiments.

In some embodiments, as illustrated in FIG. 4D, the second magnetic field generator 120 provided around the catheter 30 may be configured as an electromagnet having parallel coils.

In some embodiments, as illustrated in FIG. 4E, the second magnetic field generator 120 provided around the catheter 30 may be configured as an electromagnet having serial coils.

The second magnetic field generator 120 may be positioned along the circumference of a distal portion of the catheter 30 to generate a magnetic field that focuses accelerated electron beams to a central axis of the catheter 30. Accordingly, as the magnetic field generated by the second magnetic field generator 120 focuses the accelerated electron beams to the central axis of the catheter 30, the collision with the inner wall of the catheter 30 may be prevented when the accelerated electron beams pass through the catheter 30, and the output loss of the accelerated electron beams due to the collision with the inner wall of the catheter 30 may be prevented when the accelerated electron beams pass through the catheter 30. Here, a distal part of the catheter 30 refers to an end part positioned away from the target site among opposite ends of the catheter 30, and a proximal part of the catheter 30 means an end part positioned adjacent to the target site among opposite ends of the catheter 30. In other words, the distal part of the catheter 30 may be one end of the catheter 30 adjacent to the accelerator tube 24, and the proximal part of the catheter 30 may be the other end of the catheter 30 adjacent to the first magnetic field generator 50.

For example, the second magnetic field generator 120 may be composed of one or more magnetic bodies (not shown). The second magnetic field generator 120 may be positioned to be parallel to the central axis of the catheter 30 along the circumference of the distal part of the catheter 30, and may be implemented to generate only a magnetic field in a vector direction parallel to a direction in which accelerated electron beams pass through the catheter 30. In detail, when the second magnetic field generator 120 includes four magnetic bodies, the four magnetic bodies may be positioned symmetrically with each other by two, and N poles of the four magnetic bodies may be positioned inside the catheter such that two magnetic bodies are directed toward the other two magnetic bodies. However, in this example, the number of magnetic bodies and the placement direction of a plurality of magnetic bodies are not limited thereto. For another example, an auxiliary magnetic field generator may include a quadrupole positioned along the perimeter of the distal part of the catheter 30.

In the electron beam output device according to some embodiments, a balloon may be used to secure the inner passage of the target site. After the balloon is inserted into the inner passage of the target site, air is injected into the inside of the balloon, thereby securing the inner passage of the target site. The shape of the balloon may be a sphere, a hemisphere, a cylinder, or a square cylinder and, preferably, may be a sphere. The material of the balloon may include a material susceptible to an electron beam. Accordingly, an area susceptible to the electron beam in a balloon inserted into the inner passage of the target site may be displayed as an electron beam output area of the inner passage of the target site.

Furthermore, in an electron beam output device according to some embodiments, an injection part for jetting ink or laser to the target site may be provided to display the electron beam output area of the target site.

Moreover, an electron beam output device according to some embodiments may include a camera for photographing the target site, a lighting unit for providing illumination to the target site, and a cooling fluid supply unit for supplying cooling fluid to the target site. Here, the camera may be rotatably connected to the catheter to three-dimensionally synthesize images obtained by capturing a specific section of the target site. In addition, the camera, the lighting unit, and the cooling fluid supply unit may be provided at the end part of the catheter.

Also, an electron beam output device according to some embodiments may be provided with a wound retractor for delivering the above-described camera, lighting unit, and cooling fluid supply unit to the inner passage of the target site.

Also, an electron beam output device according to some embodiments may be provided with a monitoring unit for monitoring the location and dose of the electron beam output to the target site. This monitoring unit may be provided inside the catheter. In addition, the monitoring unit may use a radiation monitor sensor that senses the location and dose of the electron beam output to the target site.

According to some embodiments, an electron beam output device may allow electron beams to pass through a narrow area and to be irradiated to a target site by adjusting and outputting a direction of an accelerated electron beam, and may output electron beams not only to a linear output area in a direction in which the electron beam is accelerated, but also to a lateral area outside the linear output area.

Moreover, according to some embodiments, an electron beam output device may output an electron beam having an appropriate dose to each zone of the target site by adjusting and outputting the intensity of the electron beam.

Furthermore, according to some embodiments, an electron beam output device may not require a conventional electron scattering unit and a conventional applicator, thereby minimizing the loss of the electron beam generated by the electron beam generator, reducing power consumption in the electron beam generator, and improving the intensity of the electron beam generated from the electron beam generator.

Also, according to some embodiments, an electron beam output device may intensively output an electron beam to a specific area of the target site by outputting an electron beam having a pencil beam shape. Accordingly, the electron beam may be prevented from being output to normal tissue other than the target site. Comparing the inventive concept with the related art, an electron beam output device according to some embodiments may output an electron beam having a pencil beam shape in a narrow unit area (e.g., 0.5×0.5 cm²) of the target site. On the other hand, a conventional electron beam output device outputs an electron beam in a large unit area (e.g., 20×20 cm²) of the target site. Accordingly, considering that an electron beam absorption ratio of an electron beam scattering unit of the conventional electron beam output device is about 50% and a wide unit area of a target unit where an electron beam is output is 1,600 times greater than a narrow unit area of a target site where an electron beam having a pencil beam shape is output, it may be understood that an electron beam output device according to some embodiments intensively outputs an electron beam, of which the intensity is about 3,000 times higher than that of the conventional electron beam output device, to the target site.

Furthermore, according to some embodiments, an electron beam output device may output an electron beam to the target site at a dose rate of about 300 Gy/s, thereby exhibiting a FLASH effect on the target site. Here, the FLASH effect occurs when an electron beam is output to the target site at a dose rate of 40 Gy/s or higher. Considering that a general dose rate of a radiation output device is 0.1 Gy/s and a dose rate of a radiation output device is about 10 Gy/s under special conditions, an output dose rate of the electron beam in an electron beam output device according to some embodiments is 3,000 times higher than the output dose rate of general radiation. For reference, the FLASH effect may reduce the damage to normal tissue adjacent to the target site due to the output of the electron beam, and may shorten a treatment time by outputting the electron beam to the target site.

Although some embodiments are described with reference to the accompanying drawings, it will be understood by those skilled in the art to which the inventive concept pertains that the inventive concept can be carried out in other detailed forms without changing the scope and spirit or the essential features of the inventive concept. Therefore, the embodiments described above are provided by way of example in all aspects, and should be construed not to be restrictive.

While the inventive concept has been described with reference to embodiments, it will be apparent to those skilled in the art that various changes and modifications may be made without departing from the spirit and scope of the inventive concept. Therefore, it should be understood that the above embodiments are not limiting, but illustrative.

Further examples in accordance with the present disclosure are set out in the following numbered clauses:
1. An electron beam output device, comprising:
   an electron beam generator configured to generate an electron beam having a pencil beam shape;
   an electron beam accelerator configured to receive the electron beam generated by the electron beam generator, and accelerate the received electron beam;
   a catheter, through which the electron beam received from the electron beam accelerator passes, wherein the catheter is placed to be directed toward a target site, or is configured to enter an inner passage of the target site; and
   a first magnetic field generator provided in the catheter and configured to generate a magnetic field that refracts the electron beam that is outputted from the catheter.
2. The electron beam output device of clause 1, wherein the first magnetic field generator comprises a plurality of magnetic bodies arranged along a circumference of a distal end portion of the catheter.
3. The electron beam output device of clause 1, wherein the first magnetic field generator comprises a first magnetic body and a second magnetic body, which are positioned to be directed toward each other at a distal end portion of the catheter.
4. The electron beam output device of clause 3, wherein each of the first magnetic body and the second magnetic body is a permanent magnet having an arc-shaped cross section.
5. The electron beam output device of clause 3, wherein each of the first magnetic body and the second magnetic body is a pulsed electromagnet that generates a magnetic field synchronized with a pulse of the electron beam passing through the catheter.
6. The electron beam output device of clause 1, wherein the first magnetic field generator is connected with the catheter, and is configured to relatively rotate with respect to the catheter and to relatively move with respect to the catheter, and
   wherein the electron beam output device further comprises a first magnetic field generator driver configured to drive the first magnetic field generator to be relatively rotated or move with respect to the catheter.
7. The electron beam output device of clause 1, wherein the electron beam generator operates in a first output mode, in which the electron beam generator generates the electron beam with a first output intensity, or operates in a second output mode, in which the electron beam generator generates the electron beam with a second output intensity, and
   wherein the first output intensity is lower than the second output intensity.
8. The electron beam output device of clause 1, wherein the electron beam accelerator comprises:
   a radio frequency (RF) generator configured to generate a high-frequency wave; and
   an accelerator tube configured to accelerate the electron beam received from the electron beam generator by an electric field generated by the high-frequency wave transmitted from the RF generator.
9. The electron beam output device of clause 8, wherein the electron beam accelerator further comprises:
   an accelerator tube cooler configured to cool the accelerator tube.
10. The electron beam output device of clause 8, further comprising:
   a vacuum pump configured to form a vacuum in at least one of the accelerator tube, the catheter, and the first magnetic field generator.
11. The electron beam output device of clause 8, wherein the catheter is detachably connected to the accelerator tube.
12. The electron beam output device of clause 8, wherein the catheter is integrally connected to the accelerator tube.
13. The electron beam output device of clause 8, wherein the catheter is connected with the accelerator tube, and is configured to be relatively rotate with respect to the accelerator tube, and
   wherein the electron beam output device further comprises a catheter driver configured to drive the catheter to be relatively rotated with respect to the accelerator tube.
14. The electron beam output device of clause 8, wherein the catheter has an adjustable length, and
   wherein the electron beam output device further comprises a catheter driver configured to move a specific site of the catheter in a direction closer to the accelerator tube or in a direction away from the accelerator tube, such that the length of the catheter is adjusted.
15. The electron beam output device of clause 13, wherein the catheter has an adjustable length,
   wherein the catheter driver is further configured to move a specific site of the catheter in a direction closer to the accelerator tube or in a direction away from the accelerator tube, such that the length of the catheter is adjusted, and
   wherein the catheter driver first adjusts the length of the catheter to fixes a distal end of the catheter to the inner passage of the target site at a specific depth, and then, rotates the catheter to direct the distal end of the catheter toward the target site.
16. The electron beam output device of clause 1, further comprising:
   a shield configured to surround the electron beam generator and the electron beam accelerator, connected with the catheter, and configured to shield the electron beam or secondary X-ray that is leaked from the electron beam generator and the electron beam accelerator.
17. The electron beam output device of clause 1, further comprising:
   a robotic arm connected with the electron beam accelerator, and configured to move and rotate the electron beam accelerator, the catheter, and the first magnetic field generator, respectively.
18. The electron beam output device of clause 1, further comprising:
   a power supplier configured to supply power to the electron beam generator, the electron beam accelerator, and the first magnetic field generator, respectively.
19. The electron beam output device of clause 1, further comprising:
   a second magnetic field generator positioned along a circumference of a distal portion of the catheter, and configured to generate another magnetic field that focuses accelerated electron beam to a central axis of the catheter.

## Claims

1. An electron beam output device, comprising:
an electron beam generator configured to generate an electron beam having a pencil beam shape;
an electron beam accelerator configured to receive the electron beam generated by the electron beam generator and accelerate the received electron beam;
a catheter, through which the electron beam received from the electron beam accelerator passes, wherein the catheter is placed to be directed toward a target site, or is configured to enter an inner passage of the target site;
a first magnetic field generator provided in and connected with the catheter, configured to generate a magnetic field that refracts the electron beam that is outputted from the catheter, and configured to relatively rotate with respect to the catheter;
a first magnetic field generator driver configured to drive the first magnetic field generator to be relatively rotated with respect to the catheter; and
a controller configured to control the first magnetic field generator driver such that the first magnetic field generator driver adjusts an angle of the first magnetic field generator with respect to the catheter, thereby adjusting a distribution of the electron beam output from the catheter to the target site.

2. The electron beam output device of claim 1, further comprising:
a catheter driver configured to move the catheter such that a distal end portion of the catheter is positioned at a specific depth in the inner passage of the target site,
wherein the controller is configured to:
control the catheter driver such that the distal end portion of the catheter is positioned at the specific depth; and
control the first magnetic field generator driver such that the angle of the first magnetic field generator is adjusted while the distal end portion of the catheter is positioned at the specific depth.

3. The electron beam output device of claim 1,
wherein the first magnetic field generator comprises a plurality of magnetic bodies arranged along a circumference of a distal end portion of the catheter.

4. The electron beam output device of claim 3,
wherein the plurality of magnetic bodies comprise a first magnetic body and a second magnetic body, which are positioned to be directed toward each other at the distal end portion of the catheter.

5. The electron beam output device of claim 1, further comprising:
a magnetic field shield provided around the first magnetic field generator and configured to reduce external leakage of the magnetic field generated by the first magnetic field generator.

6. The electron beam output device of claim 1,
wherein the first magnetic field generator comprises a weight provided to adjust a weight distribution of the first magnetic field generator based on an output direction of the electron beam and configured to reduce vibration generated during rotation of the first magnetic field generator.

7. The electron beam output device of claim 1, further comprising:
a vacuum pump configured to form a vacuum in a channel of the catheter through which the electron beam passes.

8. The electron beam output device of claim 1,
wherein a channel of the catheter through which the electron beam passes is filled with helium.

9. The electron beam output device of claim 7 or claim 8,
wherein the electron beam accelerator comprises an accelerator tube configured to accelerate the electron beam, and
wherein no scattering foil that scatters the electron beam is provided between the accelerator tube and the catheter.

10. The electron beam output device of claim 1,
wherein the first magnetic field generator driver comprises an actuator and a driving motor.

11. The electron beam output device of claim 1, further comprising:
a second magnetic field generator positioned along a circumference of a distal portion of the catheter adjacent to the electron beam accelerator, and configured to generate another magnetic field that focuses the accelerated electron beam to a central axis of the catheter.

12. The electron beam output device of claim 1,
wherein the electron beam generator is configured to generate an electron beam having a dose rate equal to or higher than 40 Gy/s.

13. The electron beam output device of claim 1,
wherein the controller is further configured to control an intensity, dose, dose rate, and generation timing of the electron beam generated from the electron beam generator.

14. The electron beam output device of claim 1,
wherein the controller is further configured to control an intensity, direction, and generation timing of the magnetic field generated by the first magnetic field generator.

15. The electron beam output device of claim 1, further comprising:
a robotic arm connected to the electron beam accelerator and configured to move or rotate the electron beam accelerator, the catheter, and the first magnetic field generator,
wherein the controller is further configured to control the robotic arm.
